# EUROPÄISCHE PATENTANMELDUNG

(11) **EP 1 518 581 A1**
(43) Veröffentlichungstag der Anmeldung: **30.03.2005**
(21) Anmeldenummer: 04030671.4
(22) Anmeldetag: 31.12.2002
(51) Int. Cl.: A61M 25/00, B23K 26/00

(54) **Verfahren zum Verbinden eines Ballons mit einem Schaft eines Ballonkatheters**

(62) Teilanmeldung aus: 02029113.4
(71) Anmelder: Abbott Laboratories Vascular Enterprises Limited, Dublin 2 (IE)
(72) Erfinder: Quint, Bodo, 72108 Rottenburg-Seebronn (DE); Nielsen, Stefan, 72108 Rottenburg (DE)
(74) Vertreter: Schmitz, Hans-Werner, Dipl.-Ing.

(57) **Zusammenfassung**

Zur Verbesserung der Verschweißungsqualität einer Verschweißung zwischen einem Katheterballon (1) und einem Katheterschaft (2) eines Ballonkatheters (3) wird erfindungsgemäß vorgeschlagen, nach Anbringung einer Vorfixierung (4) eine Schweißenergie-Absorbier-Einrichtung (5, 5'), vorzugsweise in Form eines gefärbten Schlauches im Bereich einer Fixierungsstelle (V) anzuordnen und danach diese Schweißenergie-Absorbier-Einrichtung (5, 5') mit der Strahlungsenergie (L) zu bestrahlen, um die Verschweißung durchzuführen. Nach der Verschweißung werden die Vorfixierung (4) und die Schweißenergie-Absorbier-Einrichtung (5, 5') entfernt.

## Beschreibung

Die Erfindung betrifft ein Verfahren zum Verbinden eines Katheterballons mit einem Katheterschaft eines Ballonkatheters gemäß Anspruch 1.

Bei einem bekannten Verfahren wird diese Verbindung durch eine lokale Schweißung, beispielsweise mittels einer Laserschweißung, durchgeführt. Diese lokale Schweißung erfordert eine Lokalisierung des Lichtstrahls auf die Verbindungsstelle, wozu die Maschine zur Durchführung des bekannten Verfahrens exakt justiert bzw. die Schweißstelle exakt positioniert werden muss. Diese Justierung ist jedoch sehr aufwendig, fehleranfällig und benötigt häufig manuelle Korrektur am Gerät. Darüberhinaus ergibt sich beim Laserschweißen das Problem, daß der technische Aufwand insgesamt sehr hoch ist und im Rahmen der Erfindung durchgeführte Untersuchungen gezeigt haben, daß sich häufig eine schlechte Gleichmäßigkeit der Verschweißung ergibt. Weiterhin muss bei diesem Verfahren das Material auf die Strahlenquelle abgestimmt werden.

Es ist daher Aufgabe der vorliegenden Erfindung, ein Verfahren zum Verbinden eines Katheterballons mit einem Katheterschaft eines Ballonkatheters zu schaffen, dessen technischer Aufwand geringer ist und dessen Schweißergebnisse gegenüber dem Stand der Technik verbessert sind.

Die Lösung dieser Aufgabe erfolgt durch die Merkmale des Anspruches 1.

Das erfindungsgemäße Verfahren ergibt zunächst den Vorteil, daß beliebige Materialien für den Katheterballon und den Katheterschaft bzw. Kathetertubus verwendet werden können, da eine Anpassung an die jeweils zu verwendende Strahlenquelle über die Schweißenergie-Absorbier-Einrichtung erfolgen kann.
Die Schweißenergie-Absorbier-Einrichtung bestimmt während der Montage die genaue Positionierung, an welcher später die Schweißung erfolgt, bzw. die Energie eingekoppelt wird. Folglich muss keine aufwendige Maschinenjustierung durchgeführt werden, da die erforderliche Lokalisierung des Lichtstrahles durch diese externe Schweißenergie-Absorbier-Einrichtung erreicht wird, die im Bereich der Fixierungsstelle exakt im Bereich der gewünschten Verschweißung angeordnet werden kann. Es ist somit ein höherer Automatisierungsgrad der Schweiß-Anlage bei gleichzeitig höherer Genauigkeit und damit auch Qualität der Schweißung möglich.
Weiterhin kann zwischen Energieeinkopplung und Fixierung bzw. Abdichtung der Schweißstelle in dem gegebenen Aufbau unterschieden werden, was die Möglichkeit eines intensiven Materialflusses ermöglicht. Eine Verdrängung von Material während des Schweißvorganges und/oder eine intensive Materialmischung sind somit möglich.

Ferner ergibt sich der Vorteil, daß das erfindungsgemäße Verfahren unabhängig von der Energiequelle ist, so daß eine Vielzahl unterschiedlicher Strahlungsquellen verwendet werden kann.

Die Unteransprüche haben vorteilhafte Weiterbildungen der Erfindung zum Inhalt.

Als besonders einfache Vorfixierung kann ein Schrumpfschlauch verwendet werden, der auf die Vorfixierungsstelle aufgebracht wird und der den mit dem Katheterschaft zu verschweißenden Befestigungsabschnitt des Ballons fixieren und abdichten kann.

Als Schweißenergie-Absorbier-Einrichtung sind ebenfalls verschiedene Ausführungsformen denkbar. Eine besonders einfache Ausführungsform stellt ein gefärbter Schlauch dar, der über die Vorfixierung exakt an die Stelle aufgebracht werden kann, an der die Verschweißung zwischen Ballonkatheter und Katheterschaft erfolgen soll. In einer weiteren Ausführungsform ist bereits die Vorfixierung lokal gefärbt und dient somit als Vorfixierung und Schweißenergie-Absorbier-Einrichtung zugleich.

Eine Alternative stellt ein Stilett dar, das in den Katheterschaft eingeschoben werden kann. Auf dem Stilett kann ein gefärbter Bereich angebracht werden, der exakt an der Stelle positioniert werden kann, an der die Schweißung gewünscht ist. Die Schweißung erfolgt in diesem Fall "von innen".

Wie zuvor ausgeführt, sind eine Vielzahl unterschiedlicher Strahlungsquellen denkbar, wie beispielsweise Laserquellen, monochromatische oder polychromatische Lichtquellen oder andere elektromagnetische Strahlungsquellen. Dabei ist zu beachten, daß sich das Schweißsubstrat und die Bündeleinrichtung stark in ihrem Absorptionsverhalten unterscheiden müssen.

Weitere Einzelheiten, Merkmale und Vorteile der Erfindung ergeben sich aus nachfolgender Beschreibung und anhand der Zeichnung.

Es zeigt:
- Fig. 1: eine schematisch stark vereinfachte Darstellung eines Ballonkatheters zur Erläuterung einer ersten Ausführungsform des erfindungsgemäßen Verfahrens, und
- Fig. 2: eine der Fig. 1 entsprechende Darstellung des Ballonkatheters zur Erläuterung einer zweiten Ausführungsform des erfindungsgemäßen Verfahrens.

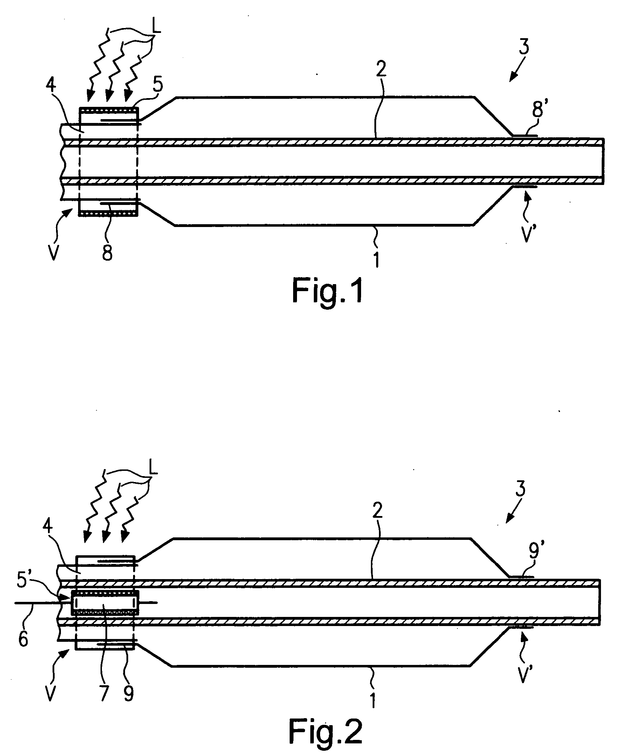

In Fig. 1 ist ein Ballonkatheter 3 in seinem proximalen Bereich dargestellt. In diesem Bereich ist ein Katheterballon 1 auf einem Katheterschaft 2 an zwei Fixierungsstellen V bzw. V' über Befestigungsabschnitte 8 bzw. 8' zu befestigen. Zur Erläuterung des erfindungsgemäßen Verfahrens wird nachfolgend auf die schematisch stark vereinfachte Darstellung im Bereich der Fixierungsstelle V Bezug genommen, deren Prinzipien jedoch auch an der Stelle V' zum Einsatz kommen können.

In Fig. 1 ist eine Ausführungsform des erfindungsgemäßen Verfahrens dargestellt, bei der zunächst eine Vorfixierung 4, hier in Form eines Schrumpfschlauches, aufgebracht wird, die den Befestigungsabschnitt 8 auf dem gewünschten Bereich des Katheterschaftes 2 befestigt/verbindet. Auf die Vorfixierung 4 wird anschließend bei der in Fig. 1 dargestellten Ausführungsform ein gefärbter Schlauch 5 aufgebracht, der eine Schweißenergie-Absorbier-Einrichtung darstellt, dessen Absorption an die Lichtquelle angepasst ist, deren Strahlengang durch den gewellten Pfeil L symbolisiert ist.

Nach Anordnung der Vorfixierung 4 und des Schlauches 5 wird die Fixierungsstelle V mit homogener Strahlung bestrahlt. Damit erfolgt eine Verschweißung des Befestigungsabschnittes 8 des Katheterballons 1 mit dem gewünschten Abschnitt des Katheterschlauches 2.

Vorteil dieses Verfahrens ist, daß bereits mit der Montage der Schweißenergie-Absorbier-Einrichtung die Lokalisation der Schweißstelle definiert ist. Die Schweißung erfolgt automatisiert in einem homogen angeregten Bereich ohne präzise Anforderungen der Position des Schweißgutes. Hierbei kann der bestrahlte Bereich auch wesentlich grösser sein als die Schweißstelle selbst.

Nach Durchführung der Verschweißung werden sowohl die Schweißenergie-Absorbier-Einrichtung als auch die Vorfixierung 4 entfernt.

In Fig. 2 ist eine alternative Ausführungsform dargestellt, bei der im Bereich der Fixierungsstelle V zur Verschweißung mit dem Befestigungsabschnitt 9 des Katheterschlauches 1 bzw. dem Befestigungsabschnitt 9' auf der distalen Seite ein Stilett 6 mit einem gefärbten Bereich 7 in den Katheterschaft 2 eingeführt wird. Das Stilett 6 und der gefärbte Bereich 7 bilden bei dieser Ausführungsform die Schweißenergie-Absorbier-Einrichtung 5'.

Nach Vorfixierung, beispielsweise durch einen Schrumpfschlauchs 4 gemäß der Ausführungsform der Fig. 1, kann wieder eine Bestrahlung mit Hilfe der Strahlungsquelle L erfolgen, so daß die zuvor anhand des Verfahrens gem. Fig. 1 erläuterte Verschweißung durch Einkopplung der Strahlungsenergie exakt im Bereich der gewünschten Verbindungsstelle erfolgt.

## Patentansprüche

1. Verfahren zum Verbinden eines Katheterballons (1) mit einem Katheterschaft (2) eines Ballonkatheters (3) mit folgenden Verfahrensschritten:
- Anordnen des Ballons (1) auf dem Katheterschaft (2);
- Anbringen einer Vorfixierung (4) zur vorläufigen Befestigung des Ballons (1) an der gewünschten Fixierungsstelle (V) des Katheterschaftes (2);
- Anordnung einer Schweißenergie-Absorbier-Einrichtung (5, 5') im Bereich der Fixierungsstelle (V) des Ballons (1) auf dem Katheterschaft (2);
- Bestrahlung der Schweißenergie-Absorbier-Einrichtung (5, 5') mit Strahlungsenergie (L); und
- Entfernen der Vorfixierung (4) und der Schweißenergie-Absorbier-Einrichtung (5, 5') nach Verschweißung des Ballons (1) mit dem Katheterschaft (2).

2. Verfahren nach Anspruch 1 **dadurch gekennzeichnet, daß** die Schweißenergie-Absorbier-Einrichtung (5, 5') bereits in die Vorfixierung (4) integriert ist.

3. Verfahren nach Anspruch 1 oder 2, **dadurch gekennzeichnet, daß** als Vorfixierung (4) ein Schrumpfschlauch auf der Fixierungsstelle (V, V') aufgebracht wird.

4. Verfahren nach einem der Ansprüche 1, 2 oder 3, **dadurch gekennzeichnet, daß** die Schweißenergie-Absorbier-Einrichtung (5, 5') einen gefärbten Bereich (7) aufweist.

5. Verfahren nach einem der Ansprüche 1, 3 oder 4, **dadurch gekennzeichnet, daß** als Schweißenergie-Absorbier-Einrichtung (5) ein gefärbter Schlauch auf der Vorfixierung (4) aufgebracht wird.

6. Verfahren nach einem der Ansprüche 1, 3 oder 4, **dadurch gekennzeichnet, daß** als Schweißenergie-Absorbier-Einrichtung (5') ein Stilett (6) mit einem gefärbten Bereich (7) in den Katheterschaft (2) im Bereich der gewünschten Fixierungsstelle (V) positioniert wird.

7. Verfahren nach einem der Ansprüche 1 bis 6, **dadurch gekennzeichnet, daß** als Licht- bzw. Strahlungsquelle (L) Laserlicht, monochromatisches Licht, polychromatisches Licht oder eine elektromagnetische Strahlungsquelle verwendet wird.

8. Verfahren nach einem der Ansprüche 1 bis 7, **dadurch gekennzeichnet, dass** die Strahlungsenergie (L) eine zumindestens nahezu homogene Verteilung aufweist.
